# FASCICULE DE BREVET EUROPEEN

(11) **EP 1 108 057 B1**
(45) Date de publication et mention de la délivrance du brevet: **06.08.2008**
(21) Numéro de dépôt: 99939479.4
(22) Date de dépôt: 23.08.1999
(51) Int. Cl.: C12Q 1/24, G01N 33/50, G01N 33/574, G01N 33/576

(54) **PROCEDE, EQUIPEMENT ET REACTIF POUR LA SEPARATION CELLULAIRE**
VERFAHREN, WERKZEUGE UND REAGENZIEN FÜR ZELLTRENNUNG
METHOD, EQUIPMENT AND REAGENT FOR CELL SEPARATION

(30) Priorité: 25.08.1998 FR 9810696
(43) Date de publication de la demande: 20.06.2001
(73) Titulaire: INSTITUT NATIONAL DE LA SANTE ET DE LA RECHERCHE MEDICALE (INSERM), 75654 Paris Cédex 13 (FR); L'ASSISTANCE PUBLIQUE - HOPITAUX DE PARIS, 75004 Paris (FR); UNIVERSITE RENE DESCARTES (PARIS V), F-75270 Paris Cédex 06 (FR)
(72) Inventeur: BISCONTE DE SAINT JULIEN, Jean-Claude, F-91640 Briis sous Forges (FR); PATERLINI, Patrizia, 75015 Paris (FR)
(74) Mandataire: Breese, Pierre
(86) Numéro de dépôt international: PCT/FR1999/001976
(87) Numéro de publication internationale: WO 2000/011210

(56) Documents cités:
- EP-A- 0 277 837
- WO-A-91/04318
- DE-A- 2 808 039
- US-A- 4 751 179
- US-A- 5 532 139
- C.H. ZIERDT ET AL.: "Development of a Lysis-Fitration Blood Culture Technique" JOURNAL OF CLINICAL MICROBIOLOGY, vol. 5, no. 1, 1977, pages 46-50, XP002101630
- VONA ET AL: "Impact of cytomorphological detection of circulating tumor cells in patients with liver cancer" HEPATHOLOGY, vol. 39, no. 3, 2004, pages 792-797,
- VONA ET AL: "Isolation by size of epithelial tumor cells, a new method for the immunomorphological and molecular characterization of circulating tumor cells" AMERICAN JOURNAL OF PATHOLOGY, vol. 156, no. 1, 2000, pages 57-63,
- WICKRAQNMASINGHE: "Blood and Bone Marrow" 1986, CHURCHILL LINVINGSTONE, , EDINBURGH LONDON MELBOURNE AND NEW YORK 3rd edition, volume 2 * pages 10-11 *

## Description

La présente invention concerne le domaine de la détection de cellules cancéreuses circulantes dans un liquide biologique, notamment dans le sang ou dans le liquide céphalo-rachidien.

L'invention concerne plus particulièrement la détection de cellules pathologiques telles que des micrométastases, pouvant diffuser spontanément après une intervention chirurgicale, en particulier l'ablation d'un organe cancéreux, ou des cellules lymphocytes infectées par des virus.

La détection précoce des micrométastases permet d'évaluer les risques de dissémination et d'apparition de cancers secondaires. La difficulté de la détection de telles micrométastases provient de la très faible quantité de cellules présentes dans un échantillon de sang.

La détection précoce de lymphocytes infectées par des virus permet de procéder à une détection indirecte et précoce de maladies virales.

Pour séparer de tels évènements rares, on a proposé dans l'art antérieur de procéder préalablement à un marquage cellulaire. En particulier, on a proposé de fixer spécifiquement les cellules cancéreuses sur des billes magnétiques. On utilise également les trieurs de cellules ou encore la méthode PCR (Polymerase Chain Reaction). Ces méthodes sont longues et peu exploitables en routine.

On a également proposé dans le brevet PCT WO9104318 un procédé de séparation de cellules vivantes différentes, telles que les cellules des îlots du pancréas et les cellules acineuses utilisant une composition comprenant une substance hydrosoluble métaboliquement inerte, qui sert à réguler la densité, l'osmolarité et le pH, une solution physiologique de stockage à froid contenant des modificateurs de viscosité augmentant la viscosité et des agents d'imperméance à action osmotique détruisant les oedèmes induits par le froid, tous contenus dans un milieu capable de maintenir les cellules viables. Les solutions sont combinées pour former une première solution ayant une densité supérieure à celle des deux types de cellules à leur état naturel mais inférieure à celle du second type de cellules après incubation dans cette solution, ainsi qu'une seconde solution ayant une densité inférieure à celle des deux types de cellules. On obtient la séparation en laissant reposer le système ou en effectuant une centrifugation.

On a également décrit dans C.H. ZIERDT "Development of a lysis-filtration blood culture technique" Journal of clinical microbiology, vol. 5 N° 1 1977, pages 46-50, un système de culture se traduisant par la rétention de cellules dans des membranes de filtration. L'échantillon biologique est soumis à l'action d'une solution de lyse. Le filtre est ensuite lavé pour permettre la récupération des cellules retenues par le filtre.

Le brevet US4751179 décrit une méthode de détermination de différentes populations de leucocytes dans le sang.

De telles solutions sont relativement difficiles à mettre en oeuvre en raison de différents artefacts propres à ces procédés. Une des difficultés provient par exemple de l'agglomération de billes et du masquage des cellules fixées sur des billes, par les billes magnétiques excédentaires. De fait, cette méthode n'est pas applicable de façon satisfaisante car elle rend difficile l'accès à la morphologie des cellules.

Le but de l'invention est de remédier à ces inconvénients en proposant un procédé facile à mettre en oeuvre, présentant une plus grande fiabilité que les méthodes de l'art antérieur et permettant la confirmation du diagnostic par l'accès à la morphologie cellulaire comme les anatomopathologistes en ont la pratique.

L'invention vise selon son acception la plus générale un procédé de séparation, et en particulier un procédé de détection de cellules rares. Elle consiste à traiter l'échantillon biologique afin de modifier de façon différentielle les cellules rares recherchées d'une part et les autres cellules d'autre part, et à assurer une migration différentielle des cellules ayant réagi avec le réactif, par rapport aux cellules n'ayant pas réagi.

A cet effet, l'invention concerne un procédé de séparation cellulaire pour l'isolation de cellules pathogéniques présentes en très faible concentration dans un échantillon de liquide biologique tel que défini dans les revendications.

Par "liquide biologique", on entendra, au sens du présent brevet, le sang et ses dérivés (plasma, sérum), ainsi que le liquide céphalo-rachidien, le liquide lymphatique et le liquide articulaire.

Par "cellules pathogéniques" on entendra, au sens du présent brevet, des cellules présentant un état anormal révélateur d'une maladie :
- des cellules épithéliales
- des cellules endothéliales
- des micrométastases
- des cellules infectées par un virus.

A titre d'exemple, l'invention permet de détecter des micrométastases dans un échantillon de sang, ou des cellules infectées par le virus de l'hépatite B ou de l'hépatite C dans un liquide biologique, ou encore des cellules infectées dans un échantillon de liquide céphalorachidien.

De préférence, on procède à la filtration de l'échantillon avec un filtre présentant des pores de 8 µm.

Selon l'invention, on procède avant l'étape de filtration à une étape de préparation de l'échantillon consistant à additionner à l'échantillon de sang un réactif de lyse des globules rouges et de durcissement différentiel des cellules étrangères au sang. La fonction de ce réactif est de protéger les cellules nuclées du sang, en préservant leur déformabilité, et de rigidifier les cellules étrangères, notamment les cellules riches en cytokératine.

Les cellules du sang peuvent ainsi, du fait de leur souplesse, traverser le filtre nonobstant une section nominale supérieure à la porosité du filtre, par déformation de la membrane. Les autres cellules sont arrêtées par le filtre, même si leurs dimensions sont comparables aux cellules cibles, en raison de la rigidification de la membrane.

Selon une variante préférée, on additionne à l'échantillon de sang un réactif composé d'EDTA, de sérum albumine bovine, de Saponine, de formaldéhyde et de PBS.

Avantageusement, le procédé comporte une étape de préparation de l'échantillon consistant à additionner à une unité d'échantillon de sang à neuf unités de réactif de lyse des globules rouges et de fixation des cellules nuclées et en ce que l'on laisse incuber ladite préparation pendant 15 minutes environ.

Selon un mode de réalisation préféré, la filtration s'effectue sous une dépression d'environ 50000 Pa.

On peut procèder à l'agitation de l'échantillon au-dessus du filtre.

L'invention concerne un procédé de séparation cellulaire pour l'isolation de cellules pathogéniques choisies dans le groupe consistant en les cellules épithéliales, les cellules endothéliales, les micrométastases et les cellules infectées par un virus, lesdites cellules pathogéniques étant présentes en très faible concentration dans un échantillon sanguin, caractérisé en ce qu'il comporte les étapes consistant à :
- additionner à l'échantillon sanguin un réactif de lyse des globules rouges et de fixation des cellules nuclées ;
- la filtration de l'échantillon sanguin à l'aide d'un filtre présentant une porosité comprise entre 5 et 10 µm ; et à
- l'isolation de cellules pathogéniques choisies dans le groupe consistant en les cellules épithéliales, les cellules endothéliales, les micrométastases et les cellules infectées par un virus.

On peut procéder à la révélation de la présence éventuelle des particules recherchées dans la partie de l'échantillon enrichie en particules recherchées.

On fait réagir un réactif modifiant la déformabilité relative des cellules recherchées par rapport à la déformabilité des cellules susceptibles de masquer les cellules recherchées.

Un réactif pour la séparation cellulaire caractérisé en ce qu'il comporte un détergent propre à dégrader la membrane lipidique des globules rouges, et un fixateur propre à durcir la membrane des cellules nuclées est décrit.

Avantageusement, le réactif comporte une partie au moins des produits suivants : l'EDTA, du BSA, de la Saponine, du Formaldéhyde et du PBS, ou des équivalents de ces produits.

Selon un mode de mise en oeuvre particulier, le réactif est constitué par 372 mg d'EDTA, 1 g de BSA, 1,75 g de Saponine, 10 ml de Formaldéhyde dosé à 37% et une quantité suffisante de PBS pour réaliser 1 1 de réactif.

Un équipement pour la détection de cellules cancéreuses dans un échantillon de sang caractérisé en ce qu'il comporte une membrane de filtration présentant une porosité comprise entre 5 et 10 µm, et de préférence une porosité d'environ 8 µm est décrit.

Selon une variante , l'équipement comporte des moyens pour créer un flux sensiblement tangentiel à la surface du filtre.

Il peut comporter en outre des moyens pour créer une dépression d'environ 50000 Pa sous le filtre.

Par ailleurs, l'équipement pour la numération de cellules isolées comporte un filtre présentant une porosité d'environ 8 microns et un moyen d'analyse d'image du filtrat.

L'équipement permet la sélection et le recueil individuel des cellules à des fins de caractérisation ultérieure (par exemple au moyen de la PCR) et il comporte un filtre de porosité adapté à la taille des cellules cibles.

L'invention permet l'application du procédé de séparation pour la détection de micrométastases dans un échantillon sanguin, pour la détection de cellules infectées par un virus dans un liquide biologique, pour la détection de cellules infectées par un virus dans un échantillon de liquide céphalorachidien ou pour la diagnostic et le suivi de rechutes de maladies cancéreuses ou virales.

L'invention permet la détection rapide d'infections chez des patients immunodéprimés par séparation cellulaire à partir d'un échantillon de liquide céphalo-rachidien; ainsi que la prévention, la détection et le suivi dans des maladies cancéreuses ou virologiques.

L'invention sera décrite dans ce qui suit à titre d'exemple non limitatif.

On prélève un échantillon de 5 ml de sang global, peu de temps après la prise de sang.

On additionne à cet échantillon 45 ml de réactif et on laisse reposer pendant 15 minutes.

Selon une variante de mise en oeuvre, le réactif est composé comme suit :
- 372 mg d'EDTA, commercialisé par la société SIGMA sous la référence E5134
- 1 g de BSA Fraction V (Sérum albumine bovine) commercialisé par la société SIGMA sous la référence E4503
- 1,75 g de Saponine commercialisé par la société FLUKA sous la référence 84510
- 10 ml de Formaldéhyde à 37% commercialisé par la société MERCK sous la référence 4003
- QSP de PBS.
   Le pH est ajusté à 7,2.
   Le PBS est préparé comme suit :
   - 8 g de chlorure de sodium
   - 0,2 g de chlorure de potassium
   - 2,3 g de di-sodium hydrogenophosphate
   - 0,2 g de Potassium dihydrogenophosphate.

Après une incubation pendant environ 15 minutes à température ambiante, on procède à la filtration de cette préparation sur une feuille de polycarbonate présentant des trous d'environ 8 µm de section. Le filtre est soumis a une dépression de l'ordre de 50000 Pa.

La filtration s'effectue à l'aide d'un équipement présentant un récipient dans lequel est déposée la préparation de sang et de réactif. Le fond de ce récipient est formé par un filtre. Selon une variante, un rotor est disposé à l'intérieur du récipient. Il forme un flux tangentiel à la surface du filtre, de façon à provoquer le détachement des cellules et éviter le colmatage du filtre en cours de filtration.

On procède ensuite au rinçage à l'aide de 5 ml d'une solution de chlorure de sodium à 0,9%, à un pH de 7.

L'impact est coloré avec 1 ml d'acridine à 0,025% à pH 6,6 (tampon citrate) ou par 1 ml d'iodure de propidium 40 µg/ml (tampo NaCl) durant 10 mn. Pendant la coloration, le filtre formant le fond du récipient est obturé pour éviter la perfusion du liquide. Le filtre est ensuite rincé par 1 ml de tampon citrate pH 3.

Les cellules ainsi isolées peuvent ensuite faire l'objet d'un comptage par un système d'imagerie, par microscopie ou par un système de comptage de photons à large champ.

Elles peuvent également être récupérées pour une amplification par PCR de l'ADN des cellules isolées.

Les cellules isolées peuvent être marquées ou faire l'objet d'une hybridation sélective par un réactif spécifique type PNA (peptide Nucléic Acids) ou par des anticorps monoclonaux.

## Revendications

1. Procédé de séparation cellulaire pour l'isolation de cellules pathogéniques choisies dans le groupe consistant en les cellules épithéliales, les cellules endothéliales, les micrométastases et les cellules infectées par un virus, lesdites cellules pathogéniques étant présentes en très faible concentration dans un échantillon sanguin, **caractérisé en ce qu'**il comporte les étapes consistant à :
- additionner à l'échantillon sanguin un réactif de lyse des globules rouges et de fixation des cellules nuclées ;
- la filtration de l'échantillon sanguin à l'aide d'un filtre présentant une porosité comprise entre 5 et 10 µm ; et à
- l'isolation de cellules pathogéniques choisies dans le groupe consistant en les cellules épithéliales, les cellules endothéliales, les micrométastases et les cellules infectées par un virus.

2. Procédé selon la revendication 1, **caractérisé en ce que** ledit filtre présente une porosité de 8 µm .

3. Procédé selon la revendication 2, **caractérisé en ce que** ladite filtration de l'échantillon sanguin est effectuée sur une feuille de polycarbonate.

4. Procédé selon l'une quelconque des revendications 1 à 3, **caractérisé en ce que** ledit réactif de lyse des globules rouges et de fixation des cellules nuclées est composé de l'acide éthylène diamine tétra acétique (EDTA), de l'albumine de sérum bovin, de la saponine, du formaldéhyde et du tampon phosphate salin (PBS).

5. Procédé selon l'une quelconque des revendications 1 à 4, **caractérisé en ce que** ladite filtration de l'échantillon sanguin est effectuée à l'aide d'un filtre soumis à une dépression de 50 000 Pa.

## Claims

1. Cell separation process for isolating pathogenic cells chosen from the group consisting of epithelial cells, endothelial cells, micrometastases and cells infected by a virus, said pathogenic cells being present in a very low concentration in a blood sample, **characterised in that** it comprises the steps consisting of:
- adding to the blood sample a reagent for lysis of the red blood cells and for fixation of the nucleated cells;
- filtration of the blood sample using a filter having a pore size of between 5 and 10 □m; and
- isolation of pathogenic cells chosen from the group consisting of epithelial cells, endothelial cells, micrometastases and cells infected by a virus.

2. Process of claim 1, **characterised in that** said filter has a pore size of 8 □m.

3. Process of claim 2, **characterised in that** said filtration of the blood sample is carried out on a polycarbonate film.

4. Process as claimed in any of claims 1 to 3,
**characterised in that** said reagent for lysis of the red blood cells and for fixation of the nucleated cells consists of ethylenediaminetetraacetic acid (EDTA), bovine serum albumin, saponin, formaldehyde and phosphate buffered saline (PBS).

5. Process as claimed in any of claims 1 to 4, **characterised in that** said filtration of the blood sample is carried out using a filter subjected to a vacuum pressure of 50,000 Pa.

## Patentansprüche

1. Zelltrennverfahren zur Isolation von pathogenen Zellen, die aus der Gruppe bestehend aus Epithelzellen, Endothelzellen, Mikrometastasen und mit einem Virus infizierten Zellen ausgewählt sind, wobei die besagten pathogenen Zellen in sehr niedriger Konzentration in einer Blutprobe vorliegen, **dadurch gekennzeichnet, dass** das Verfahren die Schritte umfasst, die aus Folgendem bestehen:
- Zugeben eines Reagens zur Lyse von roten Blutkörperchen und zur Fixierung von kernhaltigen Zellen zu der Blutprobe;
- Filtration der Blutprobe mit Hilfe eines Filters, der eine Porosität zwischen 5 und 10 µm aufweist; und
- Isolation von pathogenen Zellen, die aus der Gruppe bestehend aus Epithelzellen, Endothelzellen, Mikrometastasen und mit einem Virus infizierten Zellen ausgewählt sind.

2. Verfahren nach Anspruch 1, **dadurch gekennzeichnet, dass** der besagte Filter eine Porosität von 8 µm aufweist.

3. Verfahren nach Anspruch 2, **dadurch gekennzeichnet, dass** die besagte Filtration der Blutprobe auf einer Polycarbonatfolie durchgeführt wird.

4. Verfahren nach einem der Ansprüche 1 bis 3, **dadurch gekennzeichnet, dass** das besagte Reagens zur Lyse von roten Blutkörperchen und zur Fixierung von kernhaltigen Zellen sich aus Ethylendiamintetraessigsäure (EDTA), Rinderserumalbumin, Saponin, Formaldehyd und phosphatgepufferter Kochsalzlösung (PBS) zusammensetzt.

5. Verfahren nach einem der Ansprüche 1 bis 4, **dadurch gekennzeichnet, dass** die besagte Filtration der Blutprobe mit Hilfe eines Filters durchgeführt wird, der einem Unterdruck von 50.000 Pa unterzogen wird.
